# EUROPÄISCHE PATENTANMELDUNG

(11) **EP 2 520 313 A1**
(43) Veröffentlichungstag der Anmeldung: **07.11.2012**
(21) Anmeldenummer: 12164101.3
(22) Anmeldetag: 13.04.2012
(51) Int. Cl.: A61L 2/04, A61L 2/07, A61L 2/26, F16L 53/00

(54) **Heizbarer Flansch**

(30) Priorität: 02.05.2011 DE 102011075088
(71) Anmelder: Krones AG, 93073 Neutraubling (DE)
(72) Erfinder: Leutz, Dirk, 84061 Ergoldsbach (DE); Weinzierl, Matthias, 84174 Eching (DE); Zacharias, Jörg, 93096 Köfering (DE); Runge, Torsten, 94315 Straubing (DE)
(74) Vertreter: Grünecker, Kinkeldey, Stockmair & Schwanhäusser

(57) **Zusammenfassung**

Verfahren und Vorrichtung zur Sterilisation einer einen Dichtungssitz (6) aufweisenden Flanschverbindung, die zur Herstellung einer Verbindung zwischen produktführenden Rohrleitungen (1a, 1b) dient. Der Dichtungssitz (6) der Flanschverbindung wird durch thermischen Energieeintrag von der nicht produktführenden Seite her behandelt.

## Beschreibung

Die Erfindung betrifft ein Verfahren zur Sterilisation einer einen Dichtungssitz aufweisenden Flanschverbindung nach dem Oberbegriff des Anspruchs 1, sowie einen Flansch zum Durchführen des Verfahrens nach Anspruch 1.

Insbesondere in der Lebensmittelindustrie muss die Sterilität von produktführenden Rohrleitungen gewährleistet werden, um eine gleichbleibende Qualität der Produkte sicherzustellen. Daher werden die Rohrleitungen nach der Installation, nach Wartungsarbeiten oder bei einem Produktwechsel im produktführenden Bereich, also im Inneren der Rohrleitung, üblicherweise mit heißem Dampf über einen ausgedehnten Zeitraum von mehr als 20 Minuten behandelt. Der dafür notwendige Energiebedarf, insbesondere aber der Energieverlust der dabei entsteht, wenn die gesamte Anlage auf diese Weise sterilisiert wird, ist sehr hoch. Daher wird als weitere Möglichkeit zur Reduktion der Keimzahl oft eine chemische Desinfektion durchgeführt. Der Einsatz von Chemikalien ist zwar mit geringerem finanziellen und zeitlichen Aufwand verbunden, allerdings ist mit der chemischen Reinigung meist der Nachteil verbunden, dass der Raum hinter verbauten Dichtungen vom Desinfektionsmittel oftmals nicht erreicht wird.

Der Erfindung liegt daher die Aufgabe zugrunde, ein Verfahren und einen Flansch zur Verfügung zu stellen, der bei möglichst geringem Energieeinsatz eine Sterilisation des verbauten Dichtungssitzes ermöglicht.

Dies wird erfindungsgemäß durch ein Verfahren erreicht, bei dem eine Flanschverbindung, welche zur Herstellung einer Verbindung zwischen produktführenden Rohrleitungen einen Dichtungssitz aufweist, durch thermischen Energieeintrag von der nicht produktführenden Seite, also von außen, behandelt wird. Diese von außen eingetragene Energie ist dazu geeignet die Temperatur im Bereich des Dichtungssitzes lokal zu erhöhen. Die thermische Behandlung des Dichtungssitzes bei einer geeigneten Temperatur über eine ausreichende Zeit ermöglicht eine lokale Reduktion der Keim- und Mikroorganismenanzahl in diesem Bereich. Auf diese Weise kann auch eine energieeffiziente Sterilisation verbauter Bereiche erzielt werden, welche bei einer Reinigung mit Chemikalien, insbesondere mit flüssigen Desinfektionsmitteln, nicht erreicht werden.

Im Folgenden wird der einfacheren Darstellung wegen lediglich die abtötenden Wirkung in Bezug auf Keime beschrieben, allerdings bezieht sich die thermische Sterilisation auch auf Mikroorganismen, Viren, Sporen und Ähnliches.

Die Flanschverbindung selbst besteht hierbei aus wenigstens einem erfindungsgemäßen Flansch. Bei einer Flanschverbindung zwischen zwei produktführenden Rohrleitungen sind die Kontaktflächen der Flansche miteinander in Kontakt und die Dichtung wirkt mit dem Dichtungssitz derart zusammen, dass eine dichte Verbindung zwischen den Rohrleitungen entsteht.

Bei dem erfindungsgemäßen Flansch ist bevorzugt ein Kanal in einem Bereich nahe des Dichtungssitzes vorgesehen, durch welchen hindurch der Energieeintrag von der nicht produktführenden Außenseite ermöglicht wird.

Bei diesem erfindungsgemäßen Verfahren wird der Dichtungssitz des Flanschs im verbauten Bereich der Dichtung dadurch sterilisiert, dass von der nicht produktführenden Seite ein bevorzugt lokal auf den Dichtungssitz gerichteter Energieeintrag erfolgt, der als Temperatureintrag bei ausreichend hoher Temperatur eine keimreduzierende Wirkung besitzt. Die im Wesentlichen nur auf den Dichtungssitz bzw. den von der Dichtung verbauten Bereich konzentrierte Erwärmung ist mit einem geringen Energieaufwand verbunden.

Bevorzugt erfolgt der thermische Energieeintrag durch Zuführung eines heißen Mediums.

Das heiße Medium weist dabei bevorzugt eine Temperatur von wenigstens 120°C auf.

Vorzugsweise ist als heißes Medium Dampf oder Heißwasser vorgesehen.

In einem weiteren Schritt, welcher auch vor dem erfindungsgemäßen Verfahren erfolgen kann, kann auch der produktführende Bereich, also der innere Bereich der Rohrleitung welcher in unmittelbarem Kontakt mit dem Produkt steht, mit desinfizierend wirkenden Chemikalien beispielsweise mit flüssigem Desinfektionsmittel behandelt werden. Dazu wird das produktführende Rohrleitungssytem innenseitig mit dem Desinfektionsmittel beaufschlagt. Auf diese Weise wird die Keimzahl an der lnnsenseite der Rohre bzw. an den Tankwandungen reduziert und dadurch sterilisiert. Dadurch kann in Kombination mit der erfindungsgemäßen thermischen Behandlung der Flanschverbindung eine Sterilisation der gesamten Rohrleitung erzielt werden. Diese beiden Reinigungsschritte können unabhängig voneinander bevorzugt aber zeitgleich erfolgen.

In einer bevorzugten Ausführungsform ist an dem erfindungsgemäßen Flansch wenigstens ein Kanal vorgesehen, welcher eine erste und eine zweite Kanalöffnung, und eine dazwischen liegende Kanalmitte aufweist. Die erste Kanalöffnung ist bevorzugt an der Oberseite des Flanschs angeordnet, also an der Seite des Flanschs, welche der Kontaktfläche gegenüber liegt. Die Kanalmitte erstreckt sich räumlich über eine dünne Trennwand von diesem getrennt in einem Bereich nahe des Dichtungssitzes. Die zweite Kanalöffnung ist bevorzugt in der Kontaktfläche des Flanschs angeordnet.

In einer ersten Variante ist diese zweite Kanalöffnung dabei derart angeordnet, dass bei einer Verbindung zwischen einem ersten und einem zweiten erfindungsgemäßen Flansch, ein durchgehender Verbindungskanal entsteht, welcher eine fluchtende Verbindung zwischen der zweiten Kanalöffnung des ersten Flansches, und der entsprechenden zweiten Kanalöffnung des zweiten Flansches entstehen lässt.
In einer zweiten Variante sind die beiden Kanäle voneinander getrennt. Über den Bereich einer ersten Kanalöffnung wird dann einem der beiden Kanäle ein Heizmedium zugeführt und über den Bereich einer zweiten Kanalöffnung des anderen Kanals ebenfalls ein Heizmedium zugeführt. Ein im ersten Kanal entstehendes Kondensat wird an einer zweiten Kanalöffnung des ersten Kanals abgeführt, wohingegen im zweiten Kanal entstehendes Kondensat an einer ersten Kanalöffnung dieses zweiten Kanals abgeführt wird.

Durch diesen Verbindungskanal hindurch wird zur Sterilisation bevorzugt ein heißes Medium geleitet. Verwendet man beispielsweise heißen Dampf oder Heißwasser als energietragendes Medium und führt dieses dem Verbindungskanal zu, so steigt die Temperatur insbesondere im Bereich des Dichtungssitzes an. Dieser Temperaturanstieg wird infolge der Wärmeleitung des Flanschmaterials auch in benachbarten Bereichen des Kanals wirksam, insbesondere aber an dem nur durch die dünne Trennwand getrennten Dichtungssitz. Aufgrund der Geometrie des Kanals bzw. des Verbindungskanals wird die eingetragene Wärmeenergie am Dichtungssitz konzentriert.

Bei einer weiter bevorzugten Ausführungsform ist an dem erfindungsgemäßen Flansch ein Mittel zum Beheizen des Dichtungssitzes angeordnet. Dieses Heizmittel kann beispielsweise als Heizdraht, Heizschnur, Heizband, Heizschlauch oder als Peltier-Element ausgeführt sein. Diese Heizmittel ermöglichen damit zusammen mit weiteren Elementen wie Temperatursensoren und Spannungsquellen sowohl eine bevorzugt auf elektrischer Energiezuführung basierte Temperaturänderung als auch eine einfache Temperaturkontrolle.

Dieses Heizmittel kann bevorzugt in einen oder mehrere angeordnete Kanäle eingebracht werden. Wenigstens einer dieser Kanäle kann dabei ebenfalls eine erste Kanalöffnung aufweisen, die bevorzugt an der Oberseite des Flanschs angeordnet ist. Die Kanalmitte erstreckt sich im Bereich nahe des Dichtungssitzes. Die zweite Kanalöffnung kann ebenfalls im Kontaktbereich angeordnet sein. Alternativ kann der Kanal auch an einer beliebigen Stelle im Flansch enden, so dass keine zweite Kanalöffnung vorliegt. Das Heizmittel im Kanal füllt dabei bevorzugt den gesamten Kanalraum aus. Die elektrischen Zuleitungen zur Regelung bzw. Kontrolle der Temperatur können beispielsweise über die erste Kanalöffnung zugeführt werden.

Die Kanäle der beiden Flansche müssen in dieser Variante nicht notwendigerweise einen durchgehenden Verbindungskanal bilden.

Bei dieser Ausführung des Flanschs ist es zur Herstellung einer Flanschverbindung ausreichend, wenn nur ein Flansch erfindungsgemäß ausgebildet ist. Besteht die Flanschverbindung aus zwei erfindungsgemäßen Flanschen so kann ein gemeinsames Heizmittel oder das jeweilige Heizmittel jeweils separat oder gemeinsam mit Hilfe einer Spannungsquelle betrieben werden.

Wird die an der Spannungsquelle einstellbare Spannung des Heizmittels erhöht, so breitet sich die eingetragene elektrische Energie in Form von Wärme im Flansch, insbesondere im Bereich des Dichtungssitzes aus. Auf diese Weise kann der Bereich hinter der verbauten Dichtung gezielt beheizt und damit sterilisiert werden.

In einem zusätzlichen Reinigungsschritt kann der produktführende Bereich der Rohrleitung auch mit einer desinfizierend wirkenden Chemikalie gereinigt werden kann. Dieser Reinigungsschritt führt in Kombination mit dem erfindungsgemäßen Verfahren zur vollständigen Sterilisation der gesamten Rohrleitung.

Weitere Vorteile und Ausführungsformen ergeben sich aus den beigefügten Zeichnungen. Darin zeigen:

Fig. 1 zeigt eine Ansicht einer Rohrverbindung bestehend aus zwei erfindungsgemäßen Flanschen;

Fig. 2a zeigt eine schematische Schnittansicht des Bereichs A aus Fig. 1 in einer ersten Ausführungsform des erfindungsgemäßen Flanschs;

Fig. 2b zeigt eine schematische Schnittansicht des Bereichs A aus Fig. 1 in einer zweiten erfindungsgemäßen Ausführungsform des erfindungsgemäßen Flansches.

Fig. 3 zeigt eine schematische Schnittansicht des Bereichs A aus Fig. 1 in einer dritten Ausführungsform des erfindungsgemäßen Flanschs.

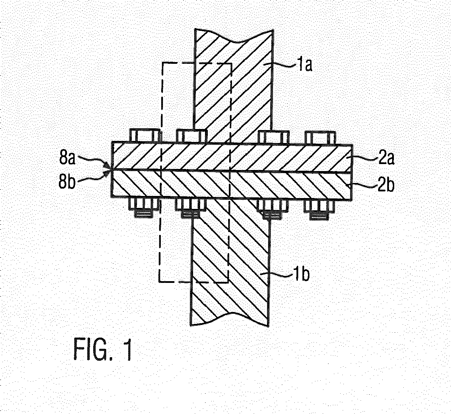

Fig. 1 zeigt zwei produktführende Rohrleitungen, welche über eine Flanschverbindung miteinander verbunden sind. Das Rohrleitungssystem umfasst dabei einen ersten Leitungsbereich **1a** mit einem ersten Flansch **2a,** sowie einen zweiten Leitungsbereich **1b** mit einem zweiten Flansch **2b.** Die Verbindung zwischen Flansch und Leitungsbereich kann hierbei eingeschraubt, verschweißt oder in sonstiger Weise erstellt sein. Die Verbindung zwischen den beiden Flanschen **2a, 2b,** wobei insbesondere die Kontaktflächen **8a, 8b** der Flansche gegeneinander gepresst werden, wird beispielsweise über ein oder mehrere Schraubmittel hergestellt.

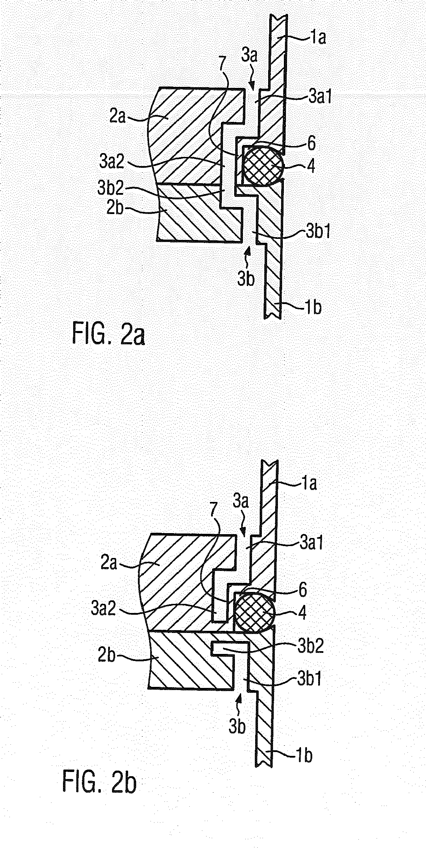

Eine erste Ausführungsform des erfindungsgemäßen Flanschs bzw. einer daraus bestehenden Flanschverbindung ist in Fig. 2a gezeigt. Darin ist eine Schnittdarstellung des markierten Bereichs A aus der Fig. 1 dargestellt. Wie hier angedeutet wirken der Dichtungssitz **6** und die Dichtung **4** derart zusammen, dass eine dichte Verbindung zwischen den Rohrleitungen **1a** und **1b** entsteht. Die Dichtung **4** kann hierbei beispielsweise das Dichtungsmaterial Viton, EPDM oder PTFE aufweisen.

In dieser Ausführungsform ist in dem ersten Flansch **2a** ein erster Kanal 3a und in dem zweiten Flanschen **2b** ein zweiter Kanal **3b** vorgesehen. Beide Kanäle verlaufen derart, dass die jeweils erste Kanalöffnung **3a1, 3b1** in dem nicht produktführenden Außenbereich an der Oberseite des Flanschs angeordnet ist und die jeweils zweite Kanalöffnung **3a2, 3b2** an den Kontaktflächen **8a, 8b** zwischen den Flanschen **2a, 2b** angeordnet sind. Die Kanäle verlaufen dabei in einem Bereich nahe des Dichtungssitzes **6.**

Im Bereich des Dichtungssitzes verläuft der Verbindungskanal möglichst nahe auf der dem Produkt abgewandten Seite der Dichtung vorbei. Der Dichtungssitz **6** selbst ist dabei bevorzugt von einer dünnen Trennwand 7 von dem Kanal **3a, 3b** getrennt. Der verbaute Bereich des Dichtungssitzes, insbesondere der Bereich hinter der Dichtung, wird oft durch eine chemische Sterilisation innerhalb des produktführenden Leitungsbereichs nicht erfasst.

Die zweite Kanalöffnung **3a2, 3b2** an der Kontaktfläche **8a, 8b** der beiden Flansche ist hierbei derart aufeinander angepasst, dass eine fluchtende Verbindung zwischen Kanal **3a** und Kanal **3b** entsteht. Auf diese Weise kann ein heißes Medium beispielsweise in die erste Kanalöffnung **3a1** des ersten Flanschs **2a** eingebracht werden und nach Durchlaufen des dichtungsnahen Bereichs an der ersten Kanalöffnung **3b1** des zweiten Flansches **2b** wieder austreten.

Als heißes Medium wird hier Dampf verwendet. Genauso könnte beispielsweise auch Heißwasser verwendet werden. Dieser heiße Dampf gelangt bevorzugt durch die erste Kanalöffnung **3a1** des ersten Flanschs **2a** in den die beiden Flansche verbindenden Verbindungskanal. Beim Durchströmen des Verbindungskanals, insbesondere im dichtungsnahen Bereich, gibt der Dampf Energie an das umgebende Material des Kanals ab und erwärmt diesen. Der Wärmetransport durch die Trennwand **7** zum angrenzenden Dichtungssitz **6** ermöglicht die gezielte, lokale Erwärmung dieses Bereichs. Hierbei ist eine möglichst dünne Trennwand **7** zur Reduzierung der Sterilisationszeit zu bevorzugen. Bei der damit verbundenen Abkühlung kondensiert der Dampf und das entstehende Kondensat verlässt den Verbindungskanal nach unten durch die erste Kanalöffnung **3b1** des zweiten Flanschs **2b.**

Der genaue Kanalverlauf des Kanals **3a** bzw. des Kanals **3b** kann dabei an das durchströmende Medium angepasst werden. Im dargestellten Fall dient heißer Dampf zur Sterilisation. Daher ist der gewählte Verlauf des Kanals **3b** im Flansch **2b** derart ausgeführt, dass das beim Durchströmen des Kanals entstehende Kondensat leicht nach unten abgeführt werden bzw. abtropfen oder zum Recycling abgeführt werden kann. Deswegen ist die erste Kanalöffnung **3b1** im zweiten Flansch **2b** im Wesentlichen vertikal orientiert, während die erste Kanalöffnung **3a1** im ersten Flansch **2a** zur leichteren Dampfzuführung bevorzugt vom Leitungsbereich **1a** weg orientiert ist.

Eine zweite Ausführungsform des erfindungsgemäßen Flansches ist als Schnittdarstellung auch in Fig. **2b** dargestellt. Dabei ist, anders als in der ersten Ausführungsform, kein durchgehender Kanal durch die beiden Flansche **2a** und **2b** vorhanden, sondern die beiden Kanäle **3a** und 3b sind voneinander getrennt.

Der erste Kanal **3a** wird von der ersten Kanalöffnung **3a1** mit einem Heizmedium, wie beispielsweise Dampf, befüllt, wobei dieses Heizmedium dann beim Durchfließen des Kanals **3a** kondensiert, so dass das dann entstehende Kondensat im Bereich der zweiten Kanalöffnung **3a2** des ersten Kanals **3a** wieder entnommen wird.

Der zweite Kanal **3b** wird im Bereich der zweiten Kanalöffnungen **3b2** mit dem gleichen oder einem anderen Heizmedium befüllt. Auch im zweiten Kanal **3b** tritt eine Kondensation auf, so dass das dort entstehende Kondensat im Bereich der ersten Kanalöffnungen **3b1** des zweiten Kanals **3b** dann entnommen wird.

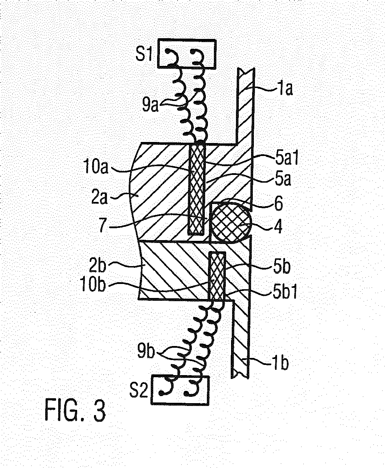

Eine dritte Ausführungsform des erfindungsgemäßen Flanschs ist als Schnittdarstellung in Fig. 3 gezeigt. Analog zu der in Fig. 2 dargestellten Ausführungsform sind auch hier ein erster Kanal **5a** im ersten Flansch **2a** und ein zweiter Kanal **5b** im zweiten Flansch **2b** vorgesehen. Genauso könnten aber auch nur in einem Flansch der Flanschverbindung ein oder mehrere Kanäle vorgesehen sein. In dieser Variante ist jeweils ein Mittel zum Beheizen des Dichtungssitzes **6,** in dieser Darstellung als Heizdraht **10a, 10b** ausgeführt, vorgesehen. Bevorzugt wird dieser Heizdraht **10a, 10b** in den jeweiligen Kanal **5a, 5b** eingebracht, wobei dieser den Kanal **5a, 5b** vollständig ausfüllt. Vorzugsweise ist der Heizdraht **10a, 10b** dabei an möglichst vielen Stellen in Kontakt mit der Kanalwand, so dass eine effiziente Übertragung der Wärmeenergie möglich ist. Zur Verbesserung dieses Kontakts zwischen Heizmittel und Kanalwand können auch Wärmeleitpasten, Wärmeleitklebstoffe oder Ähnliches verwendet werden. Neben der Verwendung eines Heizdrahtes ist auch eine Heizschnur, ein Heizband oder ein Heizschlauch denkbar.

In dieser Ausführung ist der Verlauf des Kanals **5a, 5b** derart ausgeführt, dass sich ein ausgedehnter Abschnitt des Heizmittels in der Nähe des Dichtungssitzes **6** befindet, aber räumlich durch eine dünne Trennwand **7** getrennt von diesem. Der Kanal weist hierbei lediglich eine erste Kanalöffnung **5a1, 5b1** auf, wobei der Kanal **5a, 5b** im Flansch beispielsweise in Form eines Sacklochs endet. Genauso könnte aber auch eine zweite Kanalöffnung zur Einbringung oder zum Betrieb des Heizmittels vorgesehen sein.

Die elektrischen Leitungen **9a, 9b** zum Betrieb des Heizdrahts **10a, 10b** werden durch die erste Kanalöffnung **5a1, 5b1** zugeführt und mit einer oder mehreren Spannungsquellen S1, S2 verbunden. Über die angelegte Spannung kann die Temperatur des Heizdrahts **10a, 10b** bestimmt werden.

Wird die Spannung der Spannungsquelle S1, S2 erhöht, so steigt auch die Temperatur des Heizdrahts **10a, 10b** an. Die Wärmeleitfähigkeit des umgebenden Flanschmaterials führt zu einem Temperaturanstieg des dem Kanal **5a, 5b** benachbarten Bereichs, insbesondere aber des Bereich nahe des Dichtungssitzes **6.** Die Kontrolle der angelegten Spannung ermöglicht eine gezielte Temperierung und Sterilisation des verbauten Bereichs des Dichtungssitzes **6.**

Alternativ zu der Verwendung von Kanälen, in die das Heizmittel beispielsweise eingesteckt wird, könnte das Heizmittel bereits bei der Herstellung des Flanschs eingearbeitet werden. Dies könnte beispielsweise ein Peltier-Element oder eine Heizmatte sein, welche flächig an den Kontaktflächen **8a, 8b** der Flansche **2a, 2b** derart angeordnet werden, dass diese Heizmittel den Bereich des Dichtungssitzes **6** thermisch beaufschlagen können und lediglich deren elektrische Leitungen **9a, 9b** über Kanäle nach außen geführt werden.

In den hier gezeigten Ausführungsbeispielen ist lediglich der Flansch bzw. die Flanschverbindung und das damit verbundene Verfahren zur Sterilisation des Raums hinter der verbauten Dichtung beschrieben. Separat dazu kann die Innenwand der Rohrleitung bzw. der Leitungsbereiche **1a, 1b** mit einer desinfizierend wirkenden Chemikalie beispielsweise mit einem Desinfektionsmittel sterilisiert werden.

## Patentansprüche

1. Verfahren zur Sterilisation einer einen Dichtungssitz **(6)** aufweisenden Flanschverbindung, die zur Herstellung einer Verbindung zwischen produktführenden Rohrleitungen **(1a, 1b)** dient, **dadurch gekennzeichnet, dass** der Dichtungssitz **(6)** der Flanschverbindung durch thermischen Energieeintrag von der nicht produktführenden Seite her behandelt wird.

2. Verfahren nach Anspruch 1, **dadurch gekennzeichnet, dass** der thermische Energieeintrag mittels Zuführung eines heißen Mediums erfolgt.

3. Verfahren nach Anspruch 2, **dadurch gekennzeichnet, dass** das heiße Medium eine Temperatur von wenigstens 120 °C aufweist.

4. Verfahren nach Anspruch 2 oder 3, **dadurch gekennzeichnet, dass** als heißes Medium Dampf oder Heißwasser vorgesehen ist.

5. Verfahren nach Anspruch 1, **dadurch gekennzeichnet, dass** der thermische Energieeintrag durch Verwendung eines Mittels zum Beheizen **(10a, 10b)** erfolgt.

6. Verfahren nach Anspruch 1 - 5, **dadurch gekennzeichnet, dass** in einem weiteren Schritt der produktführende Bereich der Rohrleitung **(1a, 1b)** durch Chemikalien, wie Desinfektionsmittel und/oder elektrochemisch aktiviertes Wasser (ECA-Wasser), desinfiziert wird.

7. Flansch **(2a, 2b)** zum Durchführen des Verfahrens nach Anspruch 1, **dadurch gekennzeichnet, dass** der Flansch wenigstens einen Kanal **(3a, 3b)** aufweist, welcher sich bevorzugt in einem Bereich nahe des Dichtungssitzes **(6)** erstreckt und durch den hindurch der Energieeintrag von der nicht produktführenden Seite erfolgt.

8. Flansch **(2a, 2b)** nach Anspruch 7, **dadurch gekennzeichnet, dass** der Kanal (3a, 3b) derart ausgebildet ist, dass dieser bei der Herstellung einer Flanschverbindung bestehend aus einem ersten und einem zweiten Flansch **(2a, 2b)** fluchtend vom ersten zum zweiten Flansch verläuft.

9. Flansch **(2a, 2b)** zum Durchführen des Verfahrens nach Anspruch 1, **dadurch gekennzeichnet, dass** in dem Flansch ein Mittel zum Beheizen **(10a, 10b)** vorgesehen ist.
